# EUROPEAN PATENT APPLICATION

(11) **EP 3 222 202 A1**
(43) Date of publication of application: **27.09.2017**
(21) Application number: 17162680.7
(22) Date of filing: 23.03.2017
(51) Int. Cl.: A61B 1/267, A61B 17/12, A61M 25/00, A61M 25/10

(54) **SINGLE LUMEN BALLOON DELIVERY CATHETER WITH LUMEN BYPASS AT BALLOON**

(30) Priority: 24.03.2016 US 201662312609 P; 16.03.2017 US 201715460483
(71) Applicant: Ethicon, Inc., Somerville, NJ 08876 (US)
(72) Inventor: SMITH, Daniel J., Little Egg Harbor, NJ New Jersey 08087 (US); CEDRO, Rudolph, Somerville, NJ New Jersey 08876 (US)
(74) Representative: Carpmaels & Ransford LLP

(57) **Abstract**

Systems for delivering fluid within a lumen may be provided. For example, a device (10) can include an elongated tubular structure (20) having a central opening (50) through which fluid may flow. The central opening can be defined by an open proximal end (30) and an open distal end (40). The elongated tubular structure can have an elastic section toward the open distal end. The device can also include a blocking device such as a balloon (90) within the central opening of the elongated tubular structure that can help or be configured to prevent the flow of fluid through the central opening between the open proximal end and the open distal end. The device can further include a balloon disposed on an outer surface of the elongated tubular structure. The balloon can be affixed to the outer surface of the elongated tubular structure at a proximal and/or distal attachment site.

## Description

### Field

Examples herein relate to delivery of viscous material through a delivery device, such as a catheter. Delivery can be aided by the use of a catheter including a lumen bypass and expandable balloon.

### Background

Delivery of fluids to targeted sites may be challenging, particularly when the delivery site is located in a small or narrow area, such as in a small body lumen. Delivery of fluid may be achieved in such small lumens through the use of thin, elongated catheter with a delivery port at its distal end for expelling fluid. It is even more challenging when the delivery site is located at a closed end of the lumen, due to the likelihood of the expended fluid to travel up the body lumen, past the delivery port in the catheter. Thus, the delivered fluid travels away from the intended delivery site. This is particularly difficult when a more viscous fluid, such as a gel, is delivered. Some small body lumens into which the delivery may be achieved include airways in the lung, where the fluid is to be delivered to cause lung volume reduction.

It is desirable to have a delivery system that simultaneously delivers fluid, while also blocking the body lumen, thus trapping the delivered fluid in the desired location in the blocked space. The system should also be capable of unblocking the space when the delivery catheter is to be removed, e.g., after the fluid has been delivered.

### Summary

Systems and methods for delivering fluid within a lumen may be provided. For example, a device can include an elongated tubular structure having a central opening through which fluid may flow. The central opening can be defined by an open proximal end and an open distal end. In an example, the elongated tubular structure can include or have an elastic section toward the open distal end. The device can further include a blocking device such as a balloon within the central opening of the elongated tubular structure that can help or be configured to prevent the flow of fluid through the central opening between the open proximal end and the open distal end. In an example, the device can also include a balloon disposed on an outer surface of the elongated tubular structure. The balloon can be affixed to the outer surface of the elongated tubular structure at a proximal and distal attachment site. According to an example, the device can further include an outer sheath disposed about the outer surface of the elongated tubular structure. The outer sheath can be (e.g., configured to be) sized and shaped to be slidably disposed over the balloon. The device can also be connected, in examples, to a container that can include the fluid to be dispensed through the device.

In examples, a method of delivering fluid to a target delivery site within a lumen can include inserting a distal end of a device such as the device described herein within a lumen at a target delivery site. At least a portion of an outer sheath of the device can be moved in a proximal direction such that the outer sheath does not cover an outer surface of a blocking device such as a balloon of or on the device. In examples, fluid can be forced through the central opening towards an open distal end of the device. Further, the fluid can be forced to enter the blocking device of the device, for example, causing inflation of the balloon such that an outer surface of the balloon contacts an inner surface of the lumen. In examples, the fluid can be forced to exit the device at the target delivery site. The fluid can be restricted from flowing in a proximal direction within the lumen due to a seal created by the contact of the balloon and an inner surface of the lumen.

### Brief Description of the Figures

Figure 1 shows an example of a system for delivering fluid.
Figure 2 shows the system of Figure 1 placed within a body lumen.
Figure 3 shows the system of Figure 2 with sheath retracted proximally.
Figure 4 shows the system of Figure 3 with fluid dispensed through the central lumen of the catheter.
Figure 5 shows the system of Figure 4 with fluid entering the balloon.
Figure 6 shows the system of Figure 5 with fluid flowing back into the catheter.
Figure 7 shows the system of Figure 5 with fluid flowing out through an opening in the balloon and back into the catheter.
Figure 8 shows the system of Figure 5 with fluid having been flowed through the device and into the target delivery site.
Figure 9 shows the system of Figure 8 with sheath moved distally over the balloon.
Figure 10 shows the system of Figure 9 being retracted proximally out of the lumen.
Figure 11 shows the system of Figure 10 being moved to a new target delivery site.
Figure 12 shows a system without proper relief in the balloon.
Figure 13 shows the system of Figure 12 with the balloon forming a bubbled region in the body lumen.
Figure 14 shows an alternate embodiment with an inflatable blocking device and a partially attached balloon.
Figure 15 shows the embodiment of Figure 14 with the blocking device being inflated.
Figure 16 shows an alternative embodiment of a device with an inflatable blocking device and a balloon with at least one hole.
Figure 17 shows the embedment of Figure 16 with the blocking device being partially inflated.
Figure 18 shows an alternate embodiment of a device where the balloon, partially inflated, is present in a location where there is no catheter sidewall.
Figure 19 shows the embodiment of Figure 18 with the balloon inflated.
Figure 20 shows an embodiment of a device where the balloon is present in a location where there is no catheter sidewall with the balloon deflated.
Figure 21 shows an alternate embodiment with a segment of the catheter wall which functions as an expandable member in an unexpanded configuration.
Figure 22 shows an alternate embodiment with a segment of the catheter wall which functions as an expandable member, in an expanded configuration.

### Detailed Description

Examples herein relate to devices and methods for delivering a fluid through and into a body lumen. The fluid to be delivered may be viscous or non-viscous, as will be described in greater detail below. It may be desired to deliver the fluid to a specific target site within a body lumen, while avoiding undesirable fluid flow to other regions within the same lumen. For example, it may be desired to deliver a fluid to a distal end of a lumen, such as a lung airway, but not desired to deliver that fluid to a point more proximal in the airway. Therefore, there is a need to provide a device and method to deliver the fluid to the precise location and avoid backflow of the fluid around the periphery of the delivery device and up the lumen, as well as preventing or reducing the likelihood of contamination of a bronchoscope and avoiding obscuring visualization during use.

As used herein, the term "proximal" shall generally refer to the direction that extends from the interior of the body into which the device is used out through the opening of the body into which the device is used. The term "distal" shall generally refer to the direction from outside the body into the body to the target delivery site. The term "target site" or "target delivery site" refers to the location at which fluid is intended to be delivered, which may include a region within a body lumen.

By way of example, the delivery of fluid may be achieved by inserting an elongated device into a body lumen such that the distal end of the elongated device is at or near the target delivery site within the body lumen. To be able to be fed into the body lumen, the elongated device must have an outer diameter that is equal to or smaller than the inner diameter of the body lumen into which it is inserted. As one can imagine, however, when fluid is fed from the distal end of the elongated device at the target site, fluid may flow back around the outside of the delivery device and move proximally through the body lumen between the outer diameter of the delivery device and the inner diameter of the body lumen. This is referred to as backflow. The methods and devices described herein allow for targeted delivery of fluid while avoiding undesirable backflow. The methods and devices herein also allow for complete delivery of fluid at the target site and safe and efficient removal of the delivery device after delivery is complete. The devices and methods herein also allow for delivery of an amount of fluid to the target delivery site, removal of the device from the target delivery site and movement to another target delivery site without having to completely remove the device from inside the body in which the device is placed.

In order to deliver fluid to an airway in a patient, it is useful to first feed an elongated tubular device, such as a bronchoscope, through the mouth of the patient, into the lung. Using a bronchoscope allows the feeding of the delivery devices described herein into the body and towards the airway into which fluid is intended to be delivered. It is useful to use a bronchoscope with as small a diameter as possible to allow for feeding into the body, and therefore, the devices described herein should have even smaller diameters to be capable of being fed through the bronchoscope described above. Given the intrusive nature of feeding a bronchoscope, it is useful to be able to quickly and efficiently move the delivery devices described herein from one intended target delivery site to another, without having to fully remove the device from the patient after each delivery of fluid.

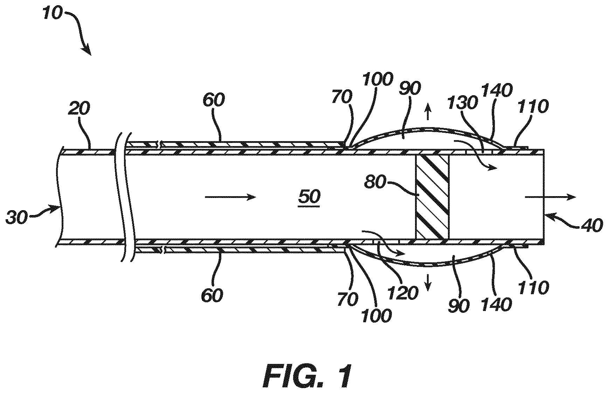

The delivery device may include a delivery assembly as generally described in Figure 1. Modifications to the components of Figure 1 are contemplated to achieve the delivery of the fluid in a similar fashion as that described herein. The delivery device 10 includes an elongated catheter 20, where the catheter 20 is a generally tubular device. The catheter 20 may be flexible enough to be manipulated by a user as it is fed into the body and towards the target delivery site. The catheter 20 body may be made of any material that is safe for use within the human body, for example, it may be made from materials such as HDPE, LDPE, silicone, rubber, vinyl, polypropylene, composite plastics, mixtures thereof. The catheter 20 may include additional structural materials, such as wire, or any other commonly used materials for catheter devices. The catheter 20 has a proximal end 30, which may extend out of the body into which the device is used during fluid delivery. The proximal end 30 may be connected or connectable to a fluid source, such as a syringe or other component suitable to hold fluid materials. In use, fluid flows into the catheter 20 at or near the proximal end 30.

The catheter 20 extends along its axial length to a distal end 40. The distal end 40 may be inserted into the patient's body into which the device is used. During use, the distal end 40 is fed through a body lumen into which fluid is to be dispensed until the distal end 40 reaches a point at or near the target delivery site. The catheter 20 desirably has a first opening (a proximal opening) at or near the proximal end 30, and has a second opening (a distal opening) at or near the distal end 40, which are in fluid communication with each other via an elongated interior opening 50. The interior opening 50 extends along the length of the catheter 20 from proximal end 30 to distal end 40. The distal opening may be referred to as the exit opening, as the fluid exits from this distal opening during delivery of fluid.

The "axis" of the catheter 20 shall refer to an imaginary line in the center of the elongated interior opening 50 from the proximal end 30 to the distal end 40, and likewise, the term "axial length" shall refer to a portion of the length as measured along a line along the axis.

The catheter 20 should be long enough to be inserted into the body into which dispensing is required, and extend through at least one body lumen to reach the target delivery site. As such, the outer diameter of the catheter 20 should be suitable to allow for insertion into the body lumen without trauma to the body lumen. In some instances, the body lumen is an airway in the lung. It may be desired to insert the catheter 20 through a bronchoscope or other such means to aide in placement or relocation of said device into the body lumen into which delivery is desired. In some instances, the bronchoscope into which the catheter 20 is inserted may be about 120 cm long. The catheter 20 may be any desired length, and may be from about 45 cm to about 180 cm, and more preferably between about 60 cm and about 120 cm, such as about 90 cm.

It is desired to use a catheter 20 having as low of an outer diameter as possible (as measured by the outer diameter taken along a line perpendicular to the central axis of the catheter). For example, the outer diameter may be from 0.5 mm to about 10 mm, or from about 1 mm to about 5 mm, or about 1 mm to about 3 mm. The outer diameter may be increased or decreased depending upon the body lumen into which it is to be inserted. The inner diameter may be slightly smaller than the outer diameter, and may be from less than about 0. 5mm to less than about 10 mm. In example embodiments, the inner diameter is about 0.025 mm to about 0.5 mm smaller than the outer diameter, the thickness of which may depend on the material used to form the catheter 20.

As noted above, the catheter 20 has a generally open interior axial opening 50 through which fluid may flow. It is desired that the catheter 20 have a single open interior 50 to allow for the flow of viscous fluid therethrough, and also to allow the catheter 20 to fit down the working channel of the bronchoscope to get as far inserted into the body lumen (such as an airway in the lung). In such instances, it may be desirable that the outer diameter of the bronchoscope gets progressively smaller as it extends into a smaller body lumen. Whether the bronchoscope's diameter tapers or remains constant, as the outer diameter of the bronchoscope gets smaller, so does its internal working channel. It is therefore one aspect of the examples herein to use a catheter 20 that has an outer diameter slightly smaller than the smallest inner diameter of the bronchoscope into which it is to be fed. This allows the device to feed fluid in the most efficient and safe way possible, while still allowing the device to be fed through the bronchoscope. In some embodiments, the device 10 may be used without the need for a bronchoscope or other feed tube. The catheter 20 inner and outer diameters may be tapered along a portion of or all of the length of the catheter 20, and if a sheath 60 is used, the sheath could be made of flexible materials to conform to catheter 20 along a portion of or all of its length.

Around the outer circumference of the catheter 20 may be an elongated sheath 60, which extends at least a portion of the axial length of the catheter 20 and surrounds the entire circumference of the catheter 20 when used. The sheath 60 may extend fully to the proximal end 30 of the catheter 20, or it may extend a portion of the axial length of the catheter 20. The sheath 60 has a distal end 70, which is located at a position more proximal than the distal end 40 of the catheter 20. The sheath 60 may be slidably secured to and in contact with the catheter 20 at one or more locations, or it may be snugly fit around the periphery of the catheter 20. The sheath 60 should be at least partially slidable along the axial length of the catheter in at least one of the distal direction or the proximal direction.

At a position within the interior opening 50 of the catheter, near the distal end 40 of the catheter 20 but not at the distal end 40, there may be a blocking device 80. The blocking device 80 may be any material or device desired, such that it creates a fluid-tight seal within the interior opening 50 of the catheter 20, thereby restricting the flow of fluid along the central opening 50 from the proximal end 30 to the distal end 40 of the catheter 20. In some embodiments, the blocking device 80 may be molded as part of the catheter 20, or in other embodiments the blocking device 80 may be an insertable device, such as a gasket, placed at a position near the distal end 40 of the catheter 20. In other aspects, the blocking device 80 may be a fillable balloon that may be fed with air or fluid via an elongated tube extending to the proximal end 30 of the catheter 20. There may be a blocking device 80 which may be controlled by fluid pressure or fluid movement, or magnetic or electric field, or spring function, which may control filling or control a physical blockage without filling.

The device 10 includes an expandable balloon 90 located on the outer surface of the catheter 20. The balloon 90 extends around the entire circumference of the catheter 20, and has an attachment to the catheter 20 at a point more proximal in axial location than the blocking device 80, and has a second attachment to the catheter 20 at a point more distal in axial location than the blocking device 80. That is, balloon 90 includes a proximal balloon attachment location 100 and a distal balloon attachment location 110 where the balloon 90 is secured to the outer surface of the catheter 20. The balloon attachment locations (100, 110) may create a fluid tight seal around the periphery of the catheter 20 outer surface. However it may also be desirable to have a tight seal at proximal location 100 and a partial seal at distal location 110. In this embodiment, attachment location 110 is connected in at least one or more locations at the distal most end of catheter 20 and at least one or more locations in the area of attachment location 110 are not tightly connected to the catheter 20, allowing a controlled amount of fluid to escape from balloon 90 distally both during fluid delivery as well as during distal movement of sheath 60 post-delivery in preparation for removal of system 10. It may be desired that the proximal balloon attachment location 100 be in between the catheter 20 body and the sheath 60, specifically at the distal end of the sheath 70.

The balloon 90 may be made from compliant material or may be made from non-compliant material. There are benefits to each. For example, compliant balloons can expand to various sizes and are made of materials such as rubber or silicone, and may have thicker walls than non-compliant balloons. Once expanded, however, compliant balloons may not return to their original size and shape. Non-compliant balloons are of a set size and shape, typically made of very thin materials, such as PET, capable of withstanding very high pressures. Non-compliant balloons may have a pre-formed shape or resemble a deflated cellophane sack, which may revert to its initial size and shape when deflated or conform to any means restraining it. In some examples, it may be useful to use a non-compliant balloon by starting with a larger size balloon that can inflate to the size of the body lumen in which it is inserted. Additionally, in examples, it may be useful to use a non-compliant balloon of a smaller diameter with a covering such as a collapsible, closed-cell foam outer covering or other outer coverings and/or a compliant balloon that can have a diameter similar to or the same as a bronchial lumen. As described below, after use, the balloon is retained in a thin flexible sheath so that the balloon does not bunch distally during insertion or removal, since it is compressed and restrained by the sheath. This allows the device, including balloon, to reliably be moved easily through the small working channel of a bronchoscope and reinserted into another body lumen for delivery of fluid.

Although either compliant balloons or non-compliant balloons are useful in the examples herein, it may be desirable to use a non-compliant balloon. More specifically, it may be, in example, desirable to use a non-compliant balloon made from PET, which provides an ability to be molded into ultra-thin walls and very precise shapes. Since PET is ultra-thin-walled, ranging from 5 to 50 microns, it is capable of producing balloons of extremely low profile. High-pressure PET balloons can be produced with diameters from 0.5 mm to 50 mm or more, in any working length, while maintaining very thin walls. They can be custom designed, they can have uniform or varying diameters along the length of the balloon and tapered ends from 1 to 90 degrees. As can be appreciated a non-compliant, thin walled balloon can have up to 150 times thinner walls than a compliant balloon.

The use of a thin-walled balloon leaves greater space within the interior opening of a bronchoscope, even if the balloon is attached to the outer surface of the catheter 20, and also allows for the balloon to be folded in one or more location between catheter 20 and sheath 60 thus allowing for a much larger balloon to be encapsulated. The unconventional use of a non-compliant balloon works well, in that it expands as much as the body lumen will allow before fluid is excreted out of the distal end 40 of the catheter 20.

The balloon 90 may be extruded, molded, or dip cast. Wall thicknesses for typical extruded balloons can be from 0.38-0.51 mm. Wall thicknesses for typical molded balloons can be 0.25-0.76 mm. Wall thicknesses for typical dip cast balloons can be 0.05-1.02 mm. In the examples herein, it may be desired that the balloon 90 have a thickness of about 10 to 40 microns or less than about 20 microns.

The catheter 20 may include an indented outer wall or region at the location of balloon 90, such that, when in a deflated state, the balloon 90 outer diameter does not extend further outward than the catheter 20 outer diameter, if secured below the outer diameter of the catheter 20, or the balloon bunching not exceed any intentional space or gap between the outer diameter of the catheter 20 and inner diameter of the sheath 60 whether secured below or on the outer diameter of the catheter 20.

The catheter 20 includes a proximal sidewall opening 120 in and through the wall of the catheter at a position more proximal in axial location than the blocking device 80, but more distal in axial location than the proximal balloon attachment location 100. Put another way, there is a proximal sidewall opening 120 in the catheter 20 wall, which is axially spaced between the blocking device 80 and the proximal balloon attachment location 100. The proximal sidewall opening 120 may be sized so as to allow or restrict the flow of fluid therethrough, and in some embodiments, the size of the proximal sidewall opening 120 is equal to or larger than a distal opening 130. In some embodiments, the proximal sidewall opening 120 has a lower resistance to the flow of fluid than the combined resistance of the distal opening 130 and/or the balloon hole(s) 140. This is desired to increase the internal balloon pressure creating a more positive seal to the body lumen. Of course, one could imagine alternate hole designs configurations if the surgical procedure was so desired. Depending upon the type of fluid to be delivered, including its viscosity and the presence or absence of solid materials within the fluid, the proximal sidewall opening 120 may be increased or decreased as needed, which may be partially dependent upon balloon hole(s) 140, if present.

Similarly, the catheter 20 may include a distal sidewall opening 130 in its wall at a position more distal in axial location than the blocking device 80, but more proximal in axial location than the distal balloon attachment location 110. Put another way, there is a distal sidewall opening 130 in the catheter 20 wall axially spaced between the blocking device 80 and the distal balloon attachment location 110. The distal sidewall opening 130 may be sized so as to allow or restrict the flow of fluid therethrough, and in desired embodiments, the size of the distal sidewall opening 130 is equal to or smaller than opening distal location. This is desired to increase the internal balloon pressure creating a more positive seal to the body lumen. Of course, one could imagine alternate hole designs configurations if the surgical procedure was so desired. Distal opening 130 and proximal sidewall opening 120 are offset from each other as measured along the central axis of the catheter 20 (with blocking device 80 therebetween), but they may also be located on different radial locations about the circumference of the catheter 20 from each other. That is, proximal sidewall opening 120 may be located about 180 degrees radially from the distal sidewall opening 130, or may be from about 90 to about 180 degrees radially offset, or from about 120 to about 150 degrees offset. Depending upon the type of fluid to be delivered, including its viscosity and the presence or absence of solid materials within the fluid, the distal sidewall opening 130 may be increased or decreased as needed, which may be partially dependent upon the balloon hole(s) 140, if present.

With the presently described configuration, fluid is allowed to flow into an opening at the proximal end 30 of the catheter 20, flowing along the open interior 50 towards the distal direction. Fluid is forced through the catheter 20 under a desired level of pressure, which will be described in greater detail below and may increase or decreased depending upon the viscosity of the fluid and the intended balloon 90 expansion level. When the fluid encounters the blocking device 80, it can no longer travel distally through the open interior 50, and is forced through the proximal sidewall opening 120, where the fluid fills the balloon 90. The balloon 90 expands to a desired radial level, thereby increasing the circumference of the delivery device 10 to a level such that the diameter of the expanded balloon 90 is greater than the outer diameter of the catheter 20 body. The radially expanded balloon 90 will contact the inner surface of the body lumen into which the device is inserted, creating a seal which is sufficient to effectively seal the body lumen to prevent unwanted leakage thereby.

The fluid then proceeds to exit the balloon 90 at the distal sidewall opening 130, where it enters the open interior 50 at a position more distal than the blocking device 80. The fluid is then permitted to exit the catheter at an opening at the distal end 40 of the device. Since the distal end 40 of the catheter 20 is inserted at or near the target delivery site, fluid expelled therefrom will be delivered at that target site.

In this aspect, the balloon 90 acts structurally as a "bypass" on the blocked catheter 20, where the balloon 90 inflates as fluid passes through. The delivery of the fluid through the catheter 20 inflates the balloon 90, causing radial expansion of the balloon 90 such that its cross-sectional diameter (as measured by a line perpendicular to the central axis of the catheter 20) is increased. It is desired that sufficient fluid, and sufficient pressure on the fluid, be introduced into the balloon 90 such that its expanded cross-sectional diameter is increased to an extent such that the balloon's outer wall contacts the interior wall of the body lumen into which it is inserted. The outer surface of the expanded balloon 90 should extend to the entire periphery of the biological lumen, thereby causing a fluid-tight seal at the contact point. As noted, the fluid-tight seal is beneficial in that it has the ability to effectively seal the body lumen to prevent unwanted leakage thereby. This may be true even if the balloon 90 has not expanded to its maximum theoretical diameter.

Notably, the expanded balloon 90 should not form the fluid tight seal at a point more distal than the distal end 40 of the catheter 20. Thus, fluid is capable of being dispensed from the catheter's distal end 40 into the body lumen at the target site. However, the formation of the fluid tight seal prevents the backflow of fluid up the body lumen in a direction towards the proximal end of the catheter 20 past the expanded balloon 90. Specifically, this keeps the fluid contained in the target delivery location in the body lumen. This is particularly important as the target area begins to fill or as it becomes harder to fill the more distal and smaller lumens. As this happens pressure distal to balloon 90 builds which in-turn increases the pressure in the balloon 90 thus automatically increasing the seal against the lumen to ensure the fluid is kept in the target area.

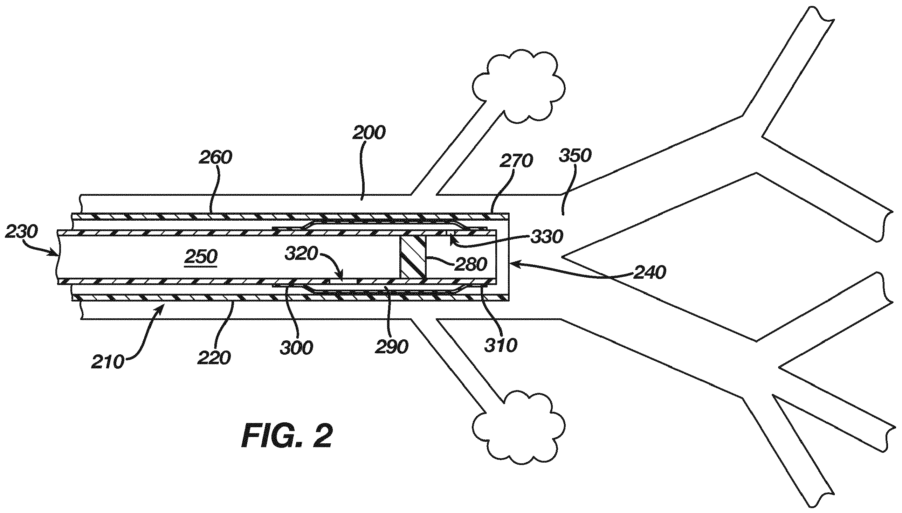

The flow of fluid through the device 10, and the subsequent inflation and deflation of the balloon 90 is seen in Figures 2 through 11. Figure 2 shows the device 210, including catheter body 220 and sheath 260 slidably secured thereto, as it is inserted into a body lumen 200, prior to the flow of fluid. The body lumen 200 in Figure 2 is a lung airway, but other body lumens may be used if desired. Figures 2-11 depict the use of a device 210 as generally described above, with reference numerals substantially similar to those described above. For example, the device (reference numeral 10) in Figure 1 correlates to device (reference numeral 210) in Figures 2-11. Similarly, the catheter in Figure 1 (20) correlates to catheter in Figures 2-11 (220), and so on. As can be seen, the distal end 240 of the device 210 is inserted at a point within the body lumen 200 at or near a target delivery site 350. The outer diameter of the catheter 220, including sheath 260, is less than the inner diameter of the body lumen 200, as well as a working channel if used. Balloon 290 is shown in its unexpanded state in Figure 2, where the balloon 290 is disposed between at least a portion of the catheter 220 and the sheath 260.

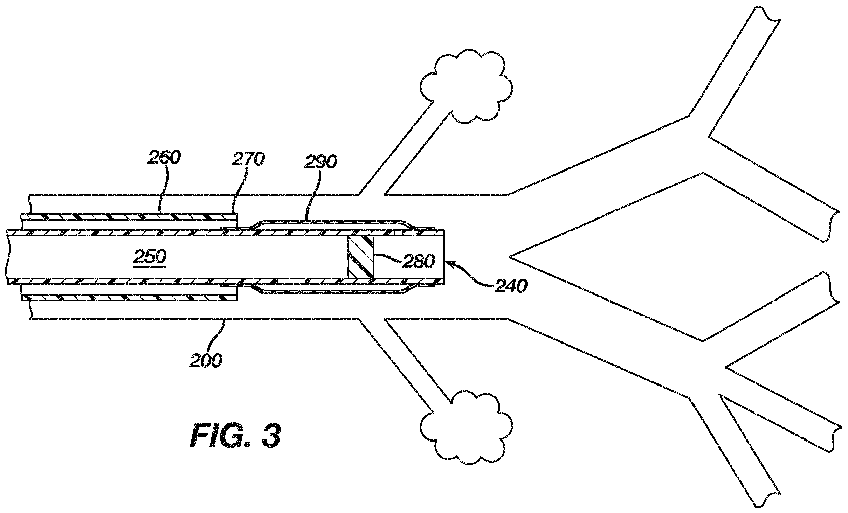

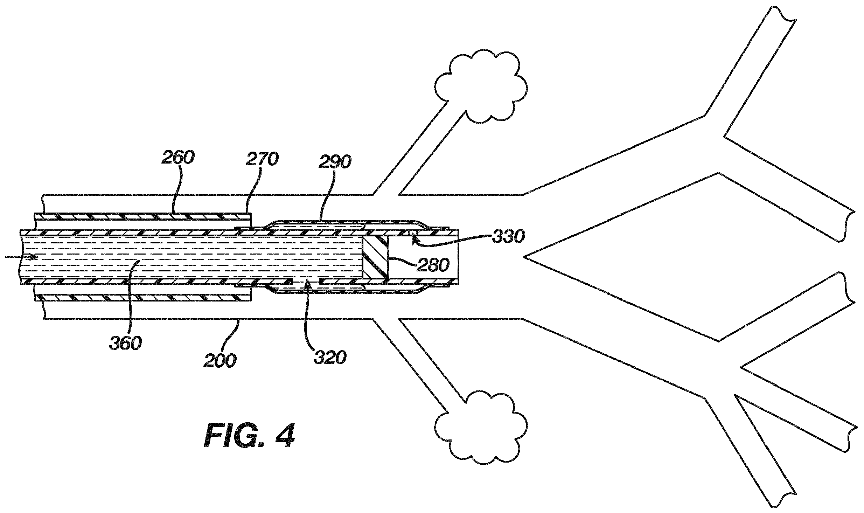

Figure 3 shows the device 210 in the same general position of Figure 2 within the body lumen 200, but shows the sheath 260 moved proximally, such that the distal end of the sheath 270 is pulled so that it is more proximal than the distal end 240 of catheter 220. It is desired that at least a part of the balloon 290, and desirably the majority of the balloon 290, is exposed and able to expand radially outward. As seen in Figure 4, the sheath 260 has been retracted as in Figure 3, and now fluid 360 (as described above) is flowed through the interior open lumen 250 of the catheter in the distal direction (e.g., towards distal end 240 and in the direction indicated by the arrow). Fluid flows through the catheter 220 until it abuts blocking component 280. As can be seen, the fluid 360 is blocked from flowing distally, and begins to flow into the balloon 290 through the proximal sidewall opening 320.

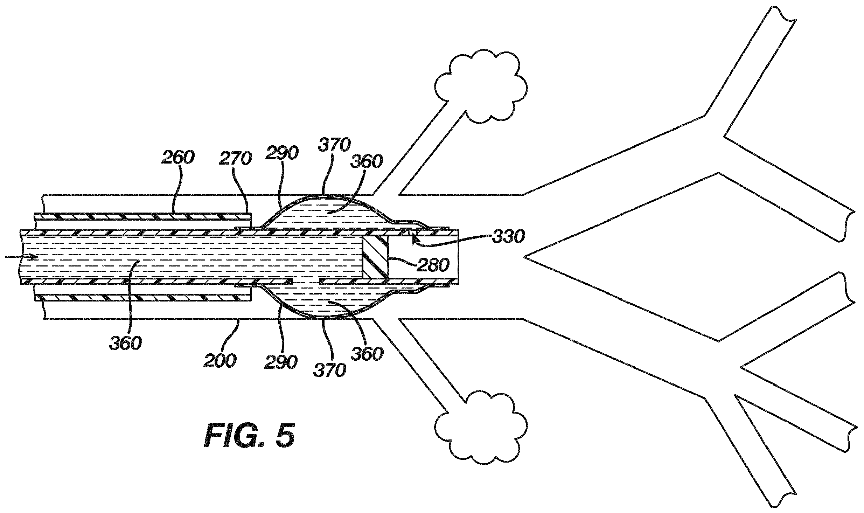

Figure 5 shows the expansion of the balloon 290 as fluid 360 flows therethrough. As also seen in Figure 5, the fluid 360 is beginning to flow through the distal sidewall opening 330. The viscosity of the fluid, the size of the opening of the distal sidewall opening 330, and the pressure exerted on the fluid 360 will help ensure that the balloon 290 is inflated to the desired level prior to fluid 360 flowing through the distal sidewall opening 330. As can be seen, the balloon 290 has expanded such that all or some of the outer surface of the balloon 290 contacts the inner surface of the body lumen 200, forming a radial seal 370 at this point of contact.

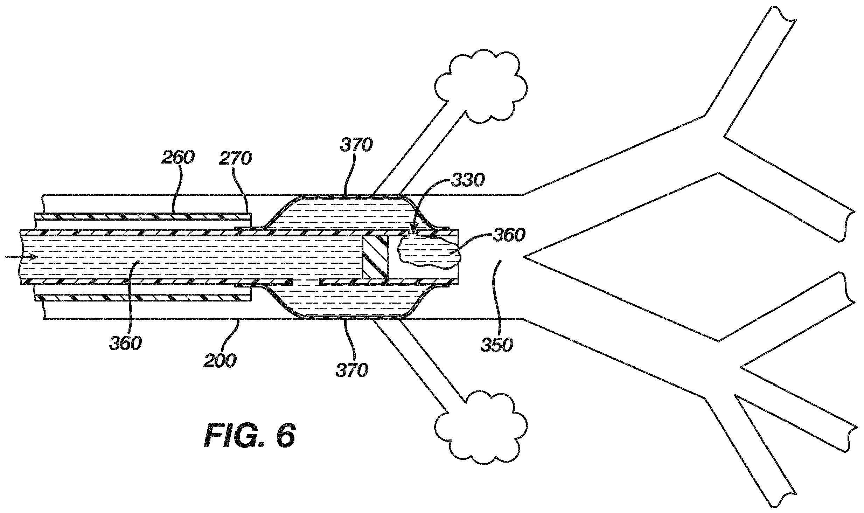

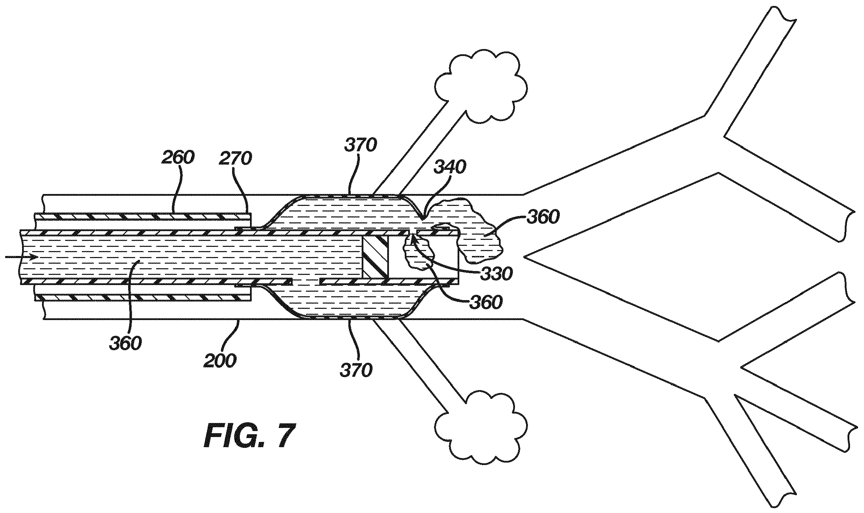

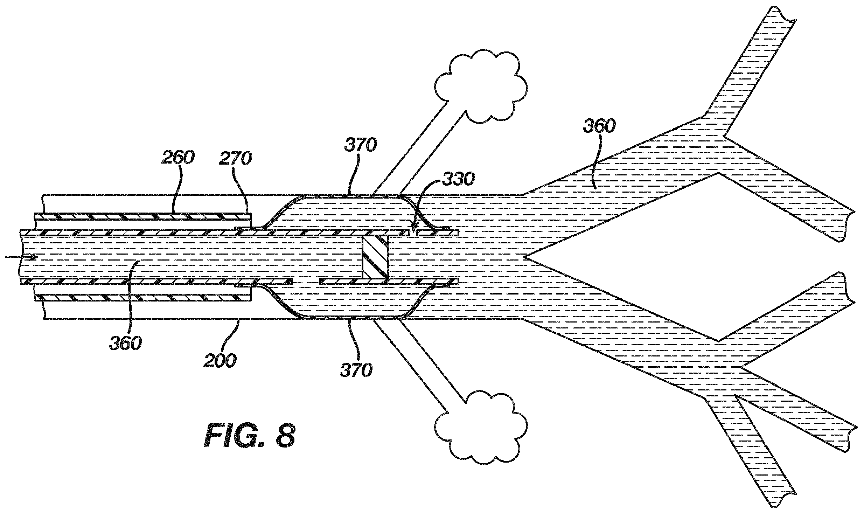

Figure 6 shows the fluid 360 dispensed into the target delivery site 350 of the body lumen 200. The fluid 360 flows around the outside of the catheter 220 within the balloon 290, forming a radial seal 370 before flowing back into the catheter 220 at the distal sidewall opening 330. From there, the fluid begins to flow out the distal end 240 of the catheter 220 and into the body lumen 200. Figure 7 shows an embodiment where the balloon 290 has perforations 340, and some of the fluid 360 being forced through balloon perforations 340 as well as into the catheter 220, such that it can be fed into the body lumen 200. Figure 8 shows the fluid 360 flowing into the target delivery site 350 in the body lumen 200. Since there is the radial seal 370 between the expanded balloon 290 and the body lumen 200, the fluid 360 is trapped within the target delivery site 350 and points within the body lumen 200 that are more distal. Fluid 360 is restricted from flowing into the body lumen proximal of the radial seal 370.

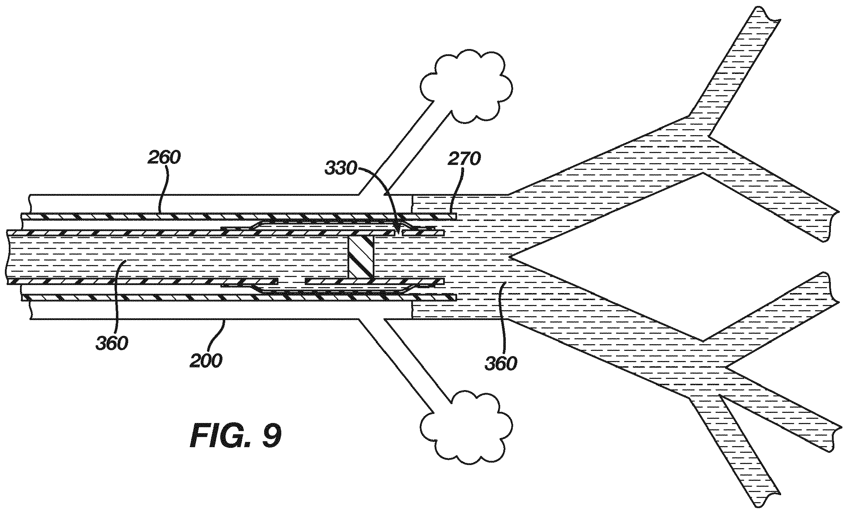

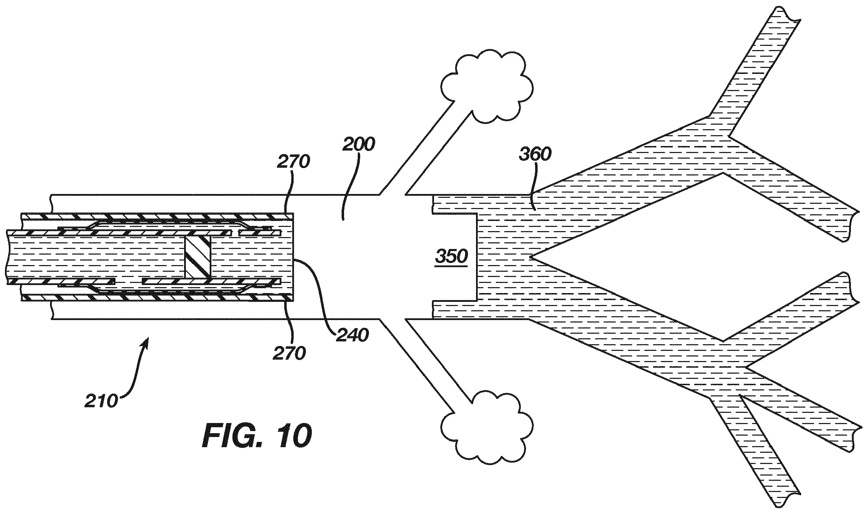

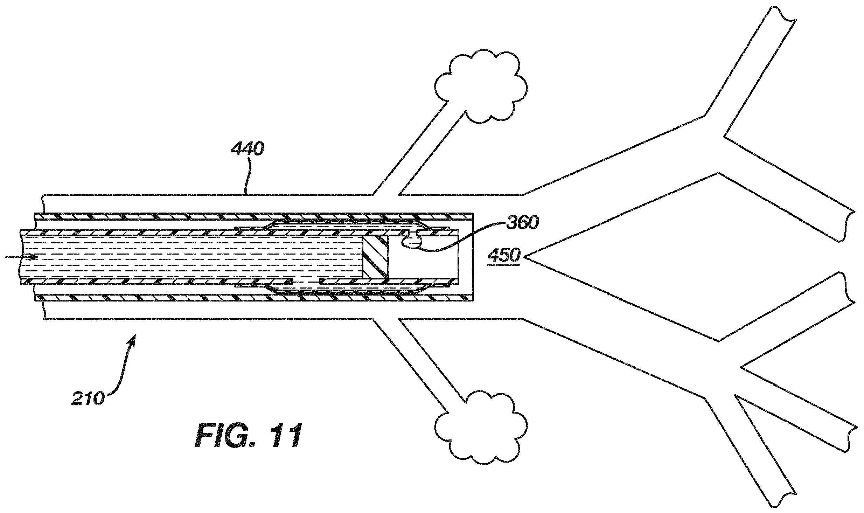

Once delivery of the fluid is completed to a sufficient degree, it is desirable to remove the delivery device 210 from the body. However, since the balloon 290 has been expanded to form the radial seal 370, in order to achieve the removal, it is desired to deflate the balloon 290. It is important to deflate the balloon 290 properly so that the balloon 290 is reduced to a diameter that is at or less than the outer diameter of the device 210 including sheath 260. Figure 9 shows the movement of the sheath 260 in the distal direction, such that the distal end 270 of the sheath 260 covers the outer surface of the balloon 290. By moving the sheath 260 distally, the user squeezes the fluid 360 out of the balloon 290 through either 330 or 340, thereby reducing the diameter of the expanded balloon 290 and removing the radial seal 370. As seen in Figure 10, the device 210 may then be retracted by pulling proximally, with the fluid 360 contained in in device 210 as well as at the target delivery site 350 and points distal to the target site 350. Figure 11 shows the device 210 being positioned within a second body lumen 440 and at a second target delivery site 450; such that the user may deliver the same fluid 360 at a different target delivery site 450 without having to fully retract the device 210 from the body of the patient. This process may be repeated as many times as desired.

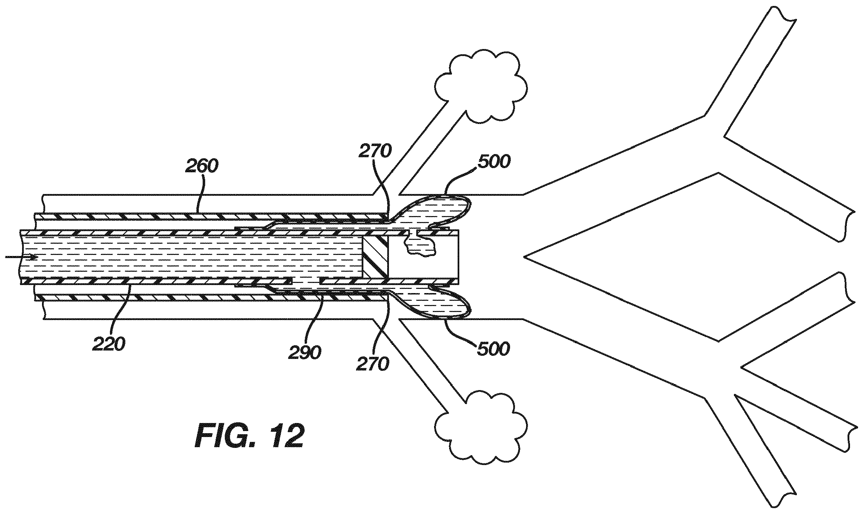

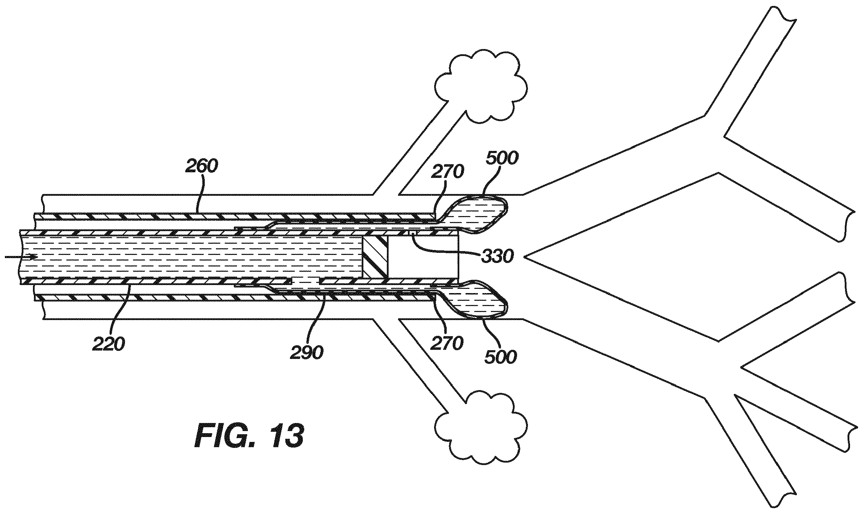

The present inventors have found, however, that simply forcing the sheath 260 distally is not suitable in some embodiments. In particular, it was found that by pushing the sheath 260 distally over the balloon 290, the balloon 290 may bubble with fluid trapped therein, forming a "donut"-type configuration at a position more distally than the distal sidewall opening 330. Figures 12 and 13 show this type of undesirable configuration. As can be seen in Figures 12 and 13, the sheath 260 has been moved distally over a portion of the balloon 290, but there is residual fluid 360 trapped in the bubbled region 500 of the balloon 290. The sheath 260 has moved distally such that it blocks the distal sidewall opening 330 with the bubbled region 500 of the balloon 290 remaining outside of the sheath 260. This configuration may risk locking the catheter to the body lumen, preventing removal. This issue problem is mainly found with highly viscous fluids that flow slowly in response to pressure created due to the distal movement of the sheath 260 over the balloon 290. This situation may not be as problematic or evident with such fluids as blood, saline or water. Trapped fluids can also result due to the use of an over-sized non-conforming balloon.

In this type of undesirable configuration, the radial diameter of the balloon 290 (at the bubbled region 500) is too large and cannot be reduced further. This expanded diameter may be too large to be retracted through the bronchoscope. Thus, the inventors have discovered that at least one or more than one perforations or relief holes 340 should be made in the balloon 290. The perforations 340 should be at a position of the balloon 290 as close to the distal end of the balloon 290 as possible, without compromising the distal balloon attachment location 310. There may be one perforation 340, or there may be two perforations 340, or there may be up to 50 to about 100 perforations 340. The number of perforations 340 may depend upon the size and spacing of such perforations 340. The perforations 340 may be disposed on the outer surface of the balloon 290, in a radial fashion or they may be linear with respect to the catheter 220. Instead of perforations or relief holes 340 being formed in the balloon 290, it is contemplated that the distal balloon attachment location 310 may have intermittent bonding points around the circumference of the catheter 220 body forming escape pathways for the fluid to be squeezed. There may be partial distal balloon attachment(s) 310, such that there is an opening or a plurality of openings at the location where the balloon 290 is secured to the catheter 220. Thus, fluid may flow through these openings, and separate perforations or holes need not be made in the balloon 290 itself.

With the perforations 340 or the alternative partial distal attachment region 310 described above, the fluid 360 is forced out of the balloon 290 into the target location 350, allowing the sheath 260 to fully encompass the balloon 290, thereby removing the radial seal 370 and reducing the diameter of the device 210 to allow the device 210 to be removed from the body lumen 200 safely and efficiently. Figure 10 shows the sheath 260 as it has been moved to fully encompass the balloon 290 and the device 210 removed proximally. In some methods, the catheter 220 may then be retracted through the bronchoscope and out of the patient. In some embodiments, the catheter 220 may be moved from a first body lumen 200 to a second body lumen 440, such as a second airway as seen in Figure 11, without having to fully remove the device 210 from the patient's body. Once moved to the second body lumen 440 (at second target delivery site 450), the sheath 260 is retracted proximally, and more fluid 360 is flowed through the catheter 220 (repeating the steps described above). In this fashion, one is capable of delivering multiple doses without removing the bronchoscope for cleaning or other purposes, ensuring visualization during the process and not prolonging the procedure.

As described earlier, the use of a non-compliant balloon is preferred due to its thin wall capability but moreover the thin walls allow the balloon to be over-sized and still be able to be gathered neatly between the catheter body (e.g., 20) and the sheath (e.g., 60). The benefits of this novel design using an over-sized balloon is counterintuitive to current thinking, and is achieved through the use of some relief mechanism at or near the distal end of the balloon. For example, as described above, there may be a plurality of perforations or relief holes, or alternatively there may be one or more openings at the balloon distal attachment site. Through the examples herein, the device uses a system that avoids the use of a typical balloon which is sealed and traps air or fluid placed in it.

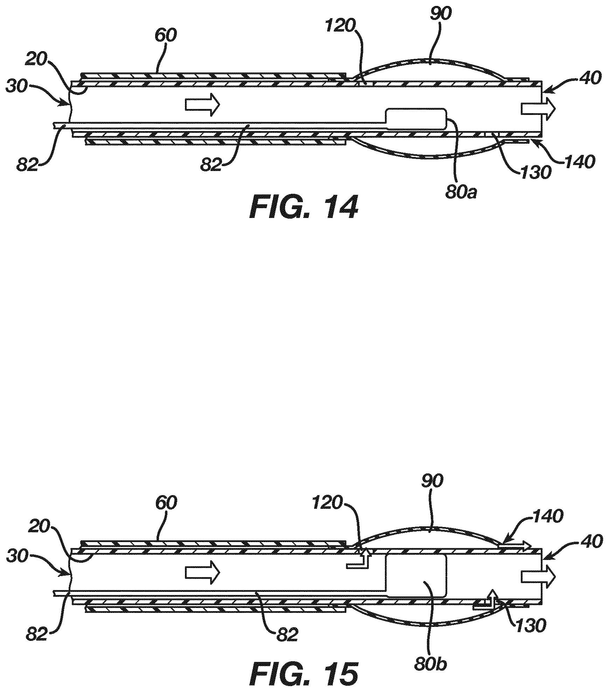

Referring now to Figures 14-17, an example is shown in a schematic cross-sectional view, whereby, as described above, the blocking device 80 may be a fillable or inflatable balloon that may be fed with air or fluid via an elongated tube or cannula 82 extending to the proximal end 30 of the catheter 20. The blocking device 80, which in this example is controlled by fluid pressure, is shown in deflated configuration 80a in Figure 14 and in fully inflated and fully blocking configuration 80b in Figure 15, whereby blocking device 80b completely blocks the lumen of catheter 20.

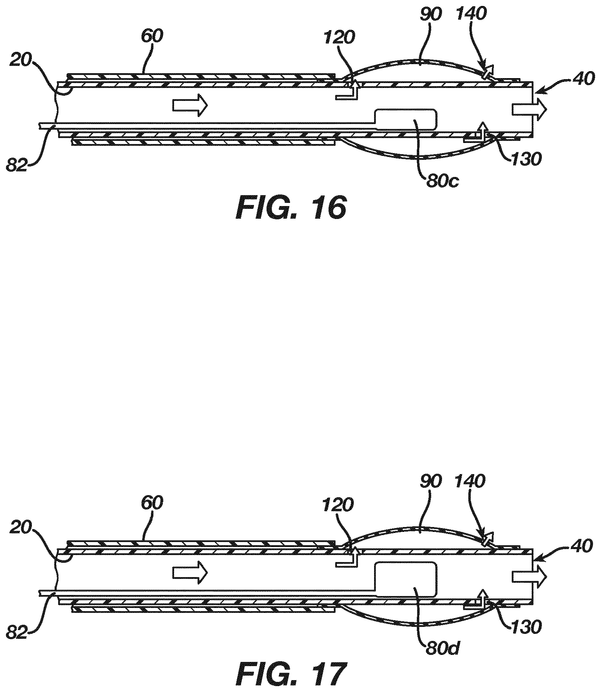

Figure 16 shows blocking device 80 in partially inflated configuration 80c at low inflation and Figure 17 shows blocking device in partially inflated configuration 80d at high inflation. Figures 15, 16, 17 also demonstrate the movement of fluid or gel through catheter 20 and balloon 90 with fully or partially inflated blocking device 80 with gel moving through proximal sidewall opening 120, distal opening 130, and balloon hole(s) 140.

Advantageously, according to one or more examples, in deflated or partially deflated configuration 80a, 80c, 80d, blocking device 80 provides for lower resistance to viscous fluid or gel passing through catheter 20 and enables tuning of gel delivery rate and pressure as well as rapid removal of excessive amount of gel by applying vacuum at the proximal end 30.

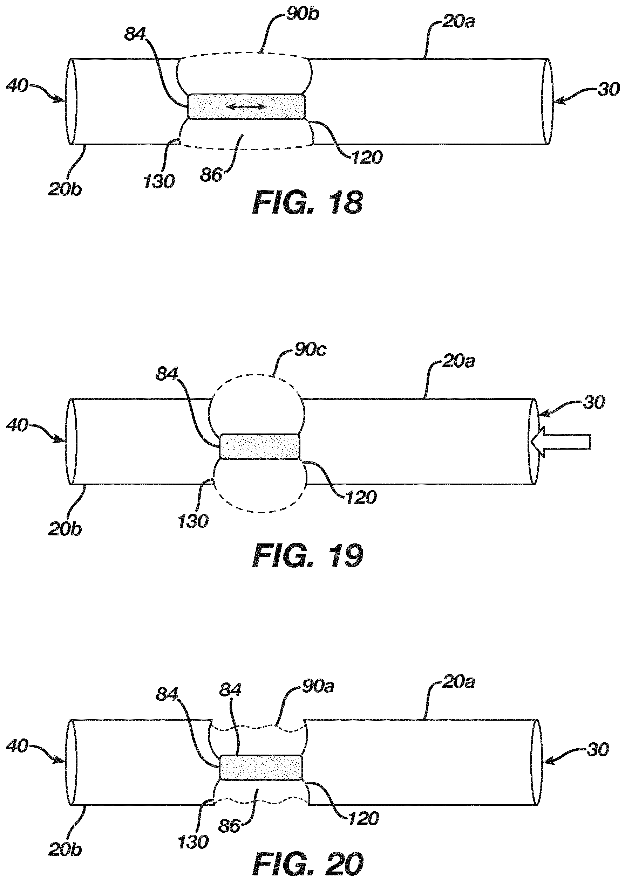

Referring now to Figures 18-20, an example is shown in a schematic cross-sectional view, whereby blocking device 84 is incorporated into the catheter 20 and comprises a spacer between a proximal portion of catheter 20a and a distal portion of catheter 20b. As shown, blocking device 84 closes proximal portion of catheter 20a and distal portion of catheter 20b and simultaneously connects these two portions which are forming catheter 20. Diameter of blocking device 84 in this embodiment is smaller than diameter of catheter 20, being from 90% to about 20% of diameter of catheter 20, such as from 80% to 30%, such as 50%. Advantageously, as can be seen in Figures 18-20, in this embodiment, a cavity 86 is formed around blocking device 84 and between proximal portion of catheter 20a and distal portion of catheter 20b. Cavity 86 is fully covered by balloon 90 which is mounted between proximal portion of catheter 20a and distal portion of catheter 20b over cavity 86 and over blocking device 84. Balloon 90 is shown in partially inflated configuration 90b in Figure 18; fully inflated configuration 90c in Figure 19; and in deflated or collapsed configuration 90a in Figure 20.

Advantageously, according to one or more examples, cavity 86 can be utilized to store balloon 90 thus rendering outside diameter of the resulting assembly closer to the outside diameter of catheter 20. Moreover, when deflating balloon 90 for withdrawal of the catheter 20 balloon 90 can be fully collapsed into cavity 86 between proximal portion of catheter 20a and distal portion of catheter 20b as seen in Figure 20 for easy withdrawal of the catheter. Such collapsing can be done with or without sheath 60 (not shown in Figures 18-20).

Optionally, blocking device 84 can be made of an elastomeric material, rendering blocking device 84 stretchable in axial directions, as schematically shown in Figure 18 by an arrow. Advantageously, according to one or more examples, excessive pressure in the balloon is then moderated by extension of blocking device 84 which decreases pressure in the balloon. Similarly, upon withdrawal of the catheter, upon encountering any resistance, pulling on proximal portion of catheter 20a will result in distension of blocking device 84 axially and further deflation of balloon 90.

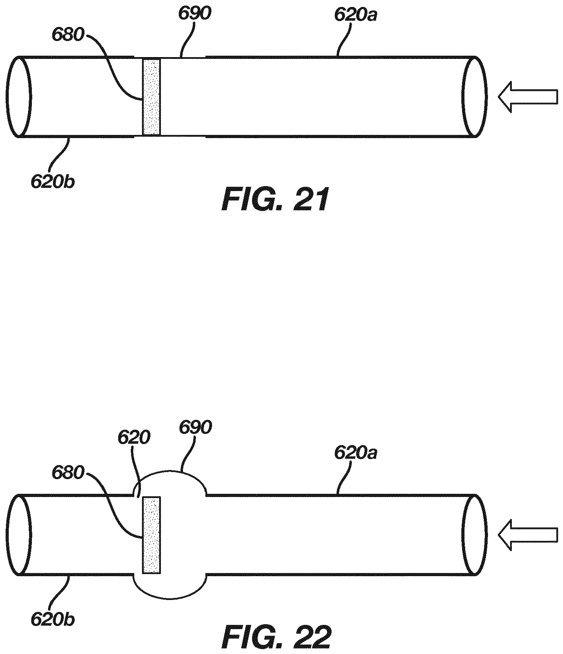

Yet another configuration capable of sealing a bodily lumen while allowing flow past a blocking device (680) within the lumen of a catheter comprises a catheter having a segment (690) made of an elastic material as shown in Figures 21 and 22. Figure 21 shows the catheter in an unexpanded configuration. The blocking element (680) is located toward the distal end of the elastic segment (690). As flow enters the catheter from the proximal end (620a), pressure will build due to the position of blockage element (680). The elastic segment (690) will expand radially as pressure builds inside of the catheter. The more pressure builds, the greater the radial expansion. Figure 22 shows the catheter in an expanded configuration. As the elastic segment (690) expands, a radial gap (620) is created. The radial gap (620) allows some pressure to be relieved and simultaneously allows flow of material from the proximal section (620a) to the distal section (620b). The relative position of the blockage element (680) with respect to the distal end of the elastic segment (690) is a primary determining factor in the relative degree of radial expansion and flow from the proximal section (620a) to the distal section (620b). The structure and configuration of blockage element (680) may be similar to the blockage elements described in other embodiments and examples.

The examples herein are further useful in that one size balloon can be used in a variety of body lumen sizes, since it has the ability to expand radially to seal off a wide range of diameters. Thus, the lumen into which the device is inserted and used may include thin lumens or larger lumens, with the ability of the balloon to not only expand suitably to create a radial seal but also deflate properly to allow for withdrawal. The radial expansion capability feature is independent of the ability to effectively seal off the targeted lumen because the balloon pressure is controlled by the size/configuration of the balloon perforations and/or the distal balloon openings which are sized based on the fluid viscosity.

It is worth noting that as the over-sized balloon diameter increases, the distal balloon attachment position to catheter may need to be shifted proximally in relationship to the distal end of the device, to ensure that when sheath is moved distally, the excess balloon shell does not extend beyond distal end of the catheter. Depending on the maximum balloon size used, the gap between the distal end of the catheter (e.g. 40) and the sheath may need to be appropriately determined to allow easy containment of the balloon between delivery sites.

The examples herein can be functional in lumens virtually as small as the size of the delivery catheter itself, or as large as about 2 to about 5 to about 10 times larger than the delivery catheter, while using a single device. The device provides controlled, safe, and effective fluid delivery in such lumens.

Any desired fluid to be delivered to the target site may be used. In some embodiments, the fluid is a gel that can fill the target site, causing the lumen to be unusable. In instances where the target site is an airway lumen, filling with certain fluid materials causes the lumen to be unusable and therefore performs lung volume reduction. The fluid may be a gel, or it may be a composite gel containing at least some powder, some solid/fiber and/or some liquid, or it may contain air or gas in any combination of powder, liquid or mixtures. As such the gel can have a wide range of viscosity between water, honey like, or even semi-solid jelly-like consistency. The viscosity of the fluid may be from about 0.01 to about 4.5 Newtons as measured and described in the viscosity measurement method below. In composite gel materials, the viscosity may be about 0.68 N. Although any fluid may be used herein, in some embodiments, the fluid may be a composite gel including a first cellulose material, such as carboxymethyl cellulose, and a powder component, such cellulose, including oxidized regenerated cellulose in the form of fibers or micro-fibers. Such combination materials that are useful include those described in Patent Application No. PCT/US15/40847, the entire contents of which are incorporated herein by reference.

The viscosity of the fluid may be measured through any desired methods, and in one method of determining viscosity, a ball measurement may be used. For example, in one suitable viscosity measurement experiment, both water and a jelly-like gel consistencies where trialed, where the jelly-like material had a viscosity of about 0.68N (0.153 lbs) when measured using a ball and socket type expression test contained in a validated Instron using a 10N load cell, and water had a viscosity of approximately 0.0N. A lower cup having a diameter of 0.750" measuring ∼3/4" deep and ball measuring 0.625" in diameter is lowered into the gel filled cup at least 15 mm (∼0.6"), at a rate of about 1 mm per second. The 0.68N force recorded resulted from expression of the gel through a small 0.06" (1/16") radial gap as the ball is pressed slowly into the gel filled lower cup. This is one test method to compare viscosities of materials, especially combination material or visco-elastic materials which can change viscosity quickly over time. Other measurement methods include the use of a traditional viscometer, movement/distance over time on an inclined plate, by way of example. Viscosity is desirably measured at room temperature, e.g., about 24 degrees C.

As noted above, the device 10 may be inserted through a bronchoscope to the lumen into which it is to be used. In some embodiments, a bronchoscope with a camera positioned proximal to the balloon may be used. In this aspect, the user (i.e., a surgeon or other clinician) can visualize further expansion of the "balloon like" shell due to pressure in the target vessel thus knowing when to stop delivering gel. Additional features could also be envisioned as part of the proximal end of the "balloon like" shell to detect and/or indicate pressure limits such as weak zones in the shell, electronic signals to detect pressures, or ink which changes color as stretched for example; as not to cause holes in the pleura of the lung lining or other tissue trauma.

It is desirable to use a delivery device that allows for the user to measure the pressure and force exerted on the fluid as it is fed through the device 10. In one embodiment, it is desired to use a balloon expander syringe which has a screw feed and pressure gauge to deliver the fluid down the catheter. This allows the user to measure and monitor the force exerted on the fluid, allowing for controlled expansion of the balloon (e.g. 90) during use.

The examples herein are advantageous in that it incorporates the use of the delivered fluid to fill the balloon 90 during use, as well as using that fluid to seal off the target area and using the balloon to deliver fluid. In other methods, air or a secondary material may be used to expand a balloon and create a radial seal. The examples herein use the different materials to be fed through the catheter and into the body lumen, thus allowing simplicity and efficacy over other methods and increasing the capability of the device to be used to deliver fluids in various lumens with varying lumen diameters.

The examples herein may be beneficial for a number of reasons, including that it eliminates the need to inflate and deflate a typical balloon catheter, such as through opening/closing of valves from a second air source or additional lumens in the catheter. In addition, it allows for a small catheter to get easily into very small vessels without trauma due to the use of over-sized balloons that can treat all lumen sizes. The examples herein can also eliminate or at least reduce the back flow of fluid past the delivery catheter end and into other airways that were not intended for treatment. Another benefit is that it eliminates the need to repeatedly remove the catheter and clean the fluid off the device, thus reducing operational and surgical time. The examples herein are also beneficial in that it needs to include a single catheter lumen to accomplish both delivery and (spherical shell) inflation. The device helps reduce the likelihood of the balloon improperly inflating, or not inflating, and also reduces the likelihood that the balloon will fail to properly deflate, thus helping prevent or reduce tissue damage or disrupting patient breathing for an extended period of time.

Since the typical patients in procedures such as lung volume reduction are quite ill, and the treated area of the lung needs to be as deflated as possible, the treatment must be quick and focused. The examples herein can aid the user in achieving a quick, focused delivery of fluid while keeping the devices and areas not intended for treatment free of fluid. Through the methods and devices described herein, such procedures can be performed more efficiently, more thoroughly, and more safely.

The examples herein further include a system for delivery of fluid, the system including the device as described above with respect to Figure 1, and a container including fluid to be dispensed. The container may be a syringe or it may be another container suitable for storing fluid allowing for the fluid to be introduced into the device, in particular, the proximal end of the device. The delivery device and container for fluid may be one unitary construction or they may be separate components. The examples herein also include a kit, including the delivery device and a container including fluid to be dispensed.

### Examples

### Example 1 - Device Without Relief Holes in a Balloon

A device including a balloon without perforations or other relief structure can be used. The fluid to be dispensed can be a composite gel having a viscosity of about 0.68N using the measurement test described above. After dispensing the fluid to the lumen, the balloon may not be capable of being deflated, and therefore, the sheath may not fully enclose the balloon and the catheter may not be removed.

It was determined that, when the fluid used is of a thick nature or has a high viscosity, it can not flow out of the small relief hole in the catheter even when forced by the distal end of the catheter sheath, since the sheath is generally located close to the catheter outer diameter in order to minimize the overall diameter of the device. Because of the close relationship of the sheath to the catheter, attempting to force the sheath distally actually increases the radial seal pressure slightly. As such, the balloon remains inflated. This is due to the gel as well as the fact the since the target area is full and pressurized. The pressure is also the same in the balloon thus it is sealed and cannot be moved. Additionally due to the viscosity, the gel could not be sucked out of the catheter to relieve the pressure.

### Example 2 - Device Including a Balloon Having Relief Holes

In an example, the balloon of Example 1 can be used, and one small 22 gauge needle hole can be punched near the catheter distal attachment point, which can (e.g., immediately) start to relieve the pressure inside the balloon. This can allow the sheath to be slid slightly further distally, which forced gel from the balloon out of the needle hole. However, once the sheath may be distal to distal sidewall opening, the remaining gel created a new seal against the lumen. The device took the configuration in Figure 13 above.

This was determined to be because both the distal sidewall opening and the initial needle hole punched in the balloon were within the distal end of sheath. A second 22 gauge needle hole can be placed or punctured in the most distal radial shell surface of the balloon, which can (e.g., immediately) start to relieve the pressure inside the balloon and allowed the sheath to be fully slid to its intended distal position. Since the balloon relief holes are distal to the distal seal area and proximal to the target area, their function can be similar to distal end of the catheter in terms of fluid delivery. According to the examples herein, it is unexpected that a balloon, which is either not sealed distally to the catheter or a balloon with relief holes, would function properly while addressing so many design and clinical issues.

It was even further surprising that the catheter itself may not need a hole or a distal catheter opening within as well as distal to the blocking device to deliver fluid, as it can be completely delivered via the perforation or interrupted balloon distal attachment site.

The invention is further defined by way of the following numbered statements:
1. A device for delivering fluid within a lumen, comprising:
   an elongated tubular structure having a central opening through which fluid may flow, the central opening being defined by an open proximal end and an open distal end;
   a balloon disposed on an outer surface of the elongated tubular structure, the balloon being affixed to the outer surface of the elongated tubular structure at a proximal attachment site and a distal attachment site;
   an outer sheath disposed about the outer surface of the elongated tubular structure, the outer sheath configured to be sized and shaped to be slidably disposed over the balloon.
2. The device of statement 1, further comprising a blocking device within the central opening of the tubular structure, preventing the flow of fluid through the central opening between the open proximal end and the open distal end.
3. The device of statement 2, wherein said blocking device comprises an inflatable balloon.
4. The device of statement 3, wherein said balloon is connected to an elongated filling cannula extending to the open proximal end.
5. The device of statement 2, wherein said blocking device comprises a spacer between a proximal portion of the tubular structure and a distal portion of the tubular structure, having diameter substantially smaller than diameter of the tubular structure.
6. The device of statement 5, wherein said blocking device is made of an elastomeric material, rendering said blocking device stretchable in axial direction.
7. The device of statement 2, wherein the blocking device is located at a point along the central axis of the device between the balloon proximal attachment site and the balloon distal attachment site.
8. The device of statement 7, wherein the elongated tubular structure has at least one proximal sidewall opening located at a position between the balloon proximal attachment site and the blocking device.
9. The device of statement 8, wherein the elongated tubular structure has at least one distal sidewall opening located at a position between the balloon distal attachment site and the blocking device.
10. The device of any one of the preceding statements, wherein the balloon includes at least one relief hole at a position near the distal attachment site.
11. The device of any one of the preceding statements, wherein the distal attachment site of the balloon includes at least one unattached portion, allowing the flow of fluid therethrough.
12. A method of delivering fluid to a target delivery site within a lumen, comprising:
   inserting a distal end of a device within a lumen at the target delivery site, the device including:
      an elongated tubular structure having a central opening through which fluid may flow, the central opening being defined by an open proximal end and an open distal end;
      a balloon disposed on an outer surface of the elongated tubular structure, the balloon being affixed to the outer surface of the elongated tubular structure at a proximal attachment site and a distal attachment site; and
      an outer sheath disposed about the outer surface of the elongated tubular structure, the outer sheath sized and shaped to be slidably disposed over the balloon;
   moving at least a portion of the outer sheath in the proximal direction, such that the outer sheath does not cover the outer surface of the balloon;
   flowing fluid through the central opening towards the open distal end;
   forcing fluid to enter the balloon, causing inflation of the balloon such that an outer surface of the balloon contacts an inner surface of the lumen; and
   forcing fluid to exit the device at the target delivery site, wherein the fluid is restricted from flowing in a proximal direction within the lumen due to a seal created by the contact of the balloon and the inner surface of the lumen.
13. The method of statement 12, wherein the device further comprises a blocking device within the central opening of the tubular structure, preventing the flow of fluid through the central opening between the open proximal end and the open distal end.
14. The method of statement 13, wherein the blocking device is located at a point along the central axis of the device between the balloon proximal attachment site and the balloon distal attachment site.
15. The method of statement 14, wherein the elongated tubular structure has at least one proximal sidewall opening located at a position between the balloon proximal attachment site and the blocking device.
16. The method of statement 15, wherein the elongated tubular structure has at least one distal sidewall opening located at a position between the balloon distal attachment site and the blocking device.
17. The method of any one of statements 12 to 16, wherein the balloon includes at least one relief hole at a position near the distal attachment site.
18. The method of any one of statements 12 to 17, wherein the distal attachment site of the balloon includes at least one unattached portion, allowing the flow of fluid therethrough.
19. The method of any one of statements 12 to 18, wherein the lumen is a body lumen.
20. The method of statement 19, wherein the body lumen is a lumen within a lung of an animal.
21. The method of any one of statements 12 to 20, wherein the fluid is a cellulose-containing fluid.
22. The method of statement 21, wherein the fluid is a carboxymethyl cellulose-containing fluid.
23. The method of any one of statements 12 to 22, wherein the fluid is a composite gel.
24. The method of statement 23, wherein the composite gel comprises a first cellulose material, such as carboxymethylcellulose, and a powder component, such as cellulose, including oxidized regenerated cellulose in the form of fibers or microfibers.
25. The method of statement 23, wherein the composite gel comprises:
   i. a first gel component, the first gel component comprising a cellulose having a molecular weight average of about 100 to about 300 kDa;
   ii. a second powder component, the second powder component comprising a dry cellulose material; and
   iii. an aqueous component.
26. The method of statement 25, wherein said aqueous component comprises water or saline.
27. The method of statement 25 or statement 26, wherein said first gel component comprises carboxymethyl cellulose.
28. The method of statement 27, wherein said first gel component comprises carboxymethyl cellulose having a molecular weight average of about 100 to about 300 kDa in an amount of about 3 to about 6 percent by weight of said first gel component.
29. The method of any one of statements 25 to 28, wherein the composite gel further comprises a salt.
30. The method of any one of statements 25 to 29, wherein said second powder component comprises a dry cellulose material.
31. The method of statement 30, wherein said second component comprises a plurality of cellulose fibers.
32. The method of statement 31, wherein said second component comprises oxidized regenerated cellulose in the form of micro-fibers.
33. The method of any one of statements 12 to 32, wherein the fluid has a viscosity of about 0.01N to about 4.5N using a ball measurement method.
34. The method of any one of statements 12 to 33, further comprising:
   moving the outer sheath in the distal direction, such that it compresses the balloon and covers the outer surface of the balloon.
35. The method of any one of statements 12 to 34, further comprising:
   withdrawing the device by pulling the device in the proximal direction, leaving the dispensed fluid at the target delivery site.
36. The method of statement 35, further comprising the step of:
   moving the device to a second lumen and positioning the distal end of the device at a second target delivery site.
37. A system for delivery of fluid, comprising:
   a fluid delivery device including:
      an elongated tubular structure having a central opening through which fluid may flow, the central opening being defined by an open proximal end and an open distal end;
      a balloon disposed on an outer surface of the elongated tubular structure, the balloon being affixed to the outer surface of the elongated tubular structure at a proximal attachment site and a distal attachment site;
      an outer sheath disposed about the outer surface of the elongated tubular structure, the outer sheath sized and shaped to be slidably disposed over the balloon; and
   a container including said fluid to be dispensed through said fluid delivery device.
38. The system of statement 37, wherein the fluid is a cellulose-containing fluid.
39. The system of statement 38, wherein the fluid is a carboxymethyl cellulose-containing fluid.
40. The system of statement 37, wherein the fluid is a composite gel.
41. The system of statement 40, wherein the composite gel comprises a first cellulose material, such as carboxymethylcellulose, and a powder component, such as cellulose, including oxidized regenerated cellulose in the form of fibers or microfibers.
42. The system of statement 41, wherein the composite gel comprises:
   i. a first gel component, the first gel component comprising a cellulose having a molecular weight average of about 100 to about 300 kDa;
   ii. a second powder component, the second powder component comprising a dry cellulose material; and
   iii. an aqueous component.
43. The system of statement 42, wherein said aqueous component comprises water or saline.
44. The system of statement 42 or statement 43, wherein said first gel component comprises carboxymethyl cellulose.
45. The system of statement 44, wherein said first gel component comprises carboxymethyl cellulose having a molecular weight average of about 100 to about 300 kDa in an amount of about 3 to about 6 percent by weight of said first gel component.
46. The system of any one of statements 42 to 45, wherein the composite gel further comprises a salt.
47. The system of any one of statements 42 to 46, wherein said second powder component comprises a dry cellulose material.
48. The system of statement 47, wherein said second component comprises a plurality of cellulose fibers.
49. The system of statement 48, wherein said second component comprises oxidized regenerated cellulose in the form of micro-fibers.
50. The system of any one of statements 37 to 49, wherein the fluid has a viscosity of about 0.01N to about 4.5N using a ball measurement method.
51. A device for delivering fluid within a lumen, comprising:
   an elongated tubular structure having a central opening through which fluid may flow, the central opening being defined by an open proximal end and an open distal end, wherein the elongated tubular structure has an elastic section toward the open distal end;
   a blocking device within the central opening of the elongated tubular structure configured to prevent the flow of fluid through the central opening between the open proximal end and the open distal end.
52. The device of statement 51, further comprising an outer sheath disposed about an outer surface of the elongated tubular structure, the outer sheath configured to be sized and shaped to be slidably disposed over the elongated tubular structure.
53. A fluid for use in a method of delivering fluid to a target delivery site within a lumen, the method comprising:
   inserting a distal end of a device within a lumen at the target delivery site, the device including:
      an elongated tubular structure having a central opening through which fluid may flow, the central opening being defined by an open proximal end and an open distal end;
      a balloon disposed on an outer surface of the elongated tubular structure, the balloon being affixed to the outer surface of the elongated tubular structure at a proximal attachment site and a distal attachment site; and
      an outer sheath disposed about the outer surface of the elongated tubular structure, the outer sheath sized and shaped to be slidably disposed over the balloon;
   moving at least a portion of the outer sheath in the proximal direction, such that the outer sheath does not cover the outer surface of the balloon;
   flowing the fluid through the central opening towards the open distal end;
   forcing the fluid to enter the balloon, causing inflation of the balloon such that an outer surface of the balloon contacts an inner surface of the lumen; and
   forcing the fluid to exit the device at the target delivery site, wherein the fluid is restricted from flowing in a proximal direction within the lumen due to a seal created by the contact of the balloon and the inner surface of the lumen.
54. A fluid for use according to statement 53, wherein the fluid is as defined in any one of statements 21 to 33.

## Claims

1. A device for delivering fluid within a lumen, comprising:
an elongated tubular structure having a central opening through which fluid may flow, the central opening being defined by an open proximal end and an open distal end;
a balloon disposed on an outer surface of the elongated tubular structure, the balloon being affixed to the outer surface of the elongated tubular structure at a proximal attachment site and a distal attachment site;
an outer sheath disposed about the outer surface of the elongated tubular structure, the outer sheath configured to be sized and shaped to be slidably disposed over the balloon.

2. The device of claim 1, further comprising a blocking device within the central opening of the tubular structure, preventing the flow of fluid through the central opening between the open proximal end and the open distal end.

3. The device of claim 2, wherein said blocking device comprises:
(i) an inflatable balloon, optionally wherein said balloon is connected to an elongated filling cannula extending to the open proximal end; or
(ii) a spacer between a proximal portion of the tubular structure and a distal portion of the tubular structure, having diameter substantially smaller than diameter of the tubular structure, optionally wherein said blocking device is made of an elastomeric material, rendering said blocking device stretchable in axial direction.

4. The device of claim 2, wherein the blocking device is located at a point along the central axis of the device between the balloon proximal attachment site and the balloon distal attachment site, optionally wherein the elongated tubular structure has at least one proximal sidewall opening located at a position between the balloon proximal attachment site and the blocking device, and further optionally wherein the elongated tubular structure has at least one distal sidewall opening located at a position between the balloon distal attachment site and the blocking device.

5. The device of any one of the preceding claims, wherein the balloon includes at least one relief hole at a position near the distal attachment site.

6. The device of any one of the preceding claims, wherein the distal attachment site of the balloon includes at least one unattached portion, allowing the flow of fluid therethrough.

7. A system for delivery of fluid, comprising:
a fluid delivery device including:
an elongated tubular structure having a central opening through which fluid may flow, the central opening being defined by an open proximal end and an open distal end;
a balloon disposed on an outer surface of the elongated tubular structure, the balloon being affixed to the outer surface of the elongated tubular structure at a proximal attachment site and a distal attachment site;
an outer sheath disposed about the outer surface of the elongated tubular structure, the outer sheath sized and shaped to be slidably disposed over the balloon; and
a container including said fluid to be dispensed through said fluid delivery device.

8. The system of claim 7, wherein the fluid is a cellulose-containing fluid, optionally wherein the fluid is a carboxymethyl cellulose-containing fluid.

9. The system of claim 7, wherein the fluid is a composite gel, optionally wherein the composite gel comprises a first cellulose material, such as carboxymethylcellulose, and a powder component, such as cellulose, including oxidized regenerated cellulose in the form of fibers or microfibers.

10. The system of claim 9, wherein the composite gel comprises:
i. a first gel component, the first gel component comprising a cellulose having a molecular weight average of about 100 to about 300 kDa;
ii. a second powder component, the second powder component comprising a dry cellulose material; and
iii. an aqueous component.

11. The system of claim 10, wherein said aqueous component comprises water or saline.

12. The system of claim 10 or claim 11, wherein said first gel component comprises carboxymethyl cellulose optionally wherein said first gel component comprises carboxymethyl cellulose having a molecular weight average of about 100 to about 300 kDa in an amount of about 3 to about 6 percent by weight of said first gel component.

13. The system of any one of claims 10 to 12, wherein the composite gel further comprises a salt.

14. The system of any one of claims 10 to 13, wherein said second powder component comprises a dry cellulose material, optionally wherein said second component comprises a plurality of cellulose fibers, optionally wherein said second component comprises oxidized regenerated cellulose in the form of micro-fibers.

15. The system of any one of claims 7 to 14, wherein the fluid has a viscosity of about 0.01N to about 4.5N using a ball measurement method.
